# EUROPEAN PATENT SPECIFICATION

(11) **EP 0 472 490 B1**
(45) Date of publication and mention of the grant of the patent: **30.11.1994**
(21) Application number: 91630050.2
(22) Date of filing: 13.08.1991
(51) Int. Cl.: G01J 5/04, G01K 1/08, A61B 5/00

(54) **Unitary probe cover**
Einteilige Sondenabdeckung
Couverture unitaire pour une sonde

(30) Priority: 24.08.1990 US 573382
(43) Date of publication of application: 26.02.1992
(73) Proprietor: THERMOSCAN INC., San Diego, California 92121 (US)
(72) Inventor: Brown, Joseph P., Valley Center, California 92082 (US); Howe, Randall R., Greeley, Colorado 80634 (US)
(74) Representative: Weydert, Robert

(56) References cited:
- US-A- 3 949 740
- US-A- 4 159 766
- US-A- 4 662 360
- US-A- 4 784 149
- US-A- 4 790 324
- US-A- 4 854 730
- US-A- 4 911 559
- US-A- 4 932 789

## Description

The present invention relates generally to probe covers. More particularly, the present invention pertains to sanitary covers for the probes or specula of medical instruments. The present invention is particularly, but not exclusively, useful as a sanitary cover for the probe of a thermometer which measures temperatures of the human body by detecting and analyzing infrared (IR) radiation from a surface of the body, such as in the ear canal.

It is well known that an accurate reading of the temperature of a human body is helpful, if not essential, for the diagnosis and monitoring of numerous ailments. Indeed, several types of thermometers have been developed for these purposes. For instance, one type of reusable thermometer which is being increasingly used is the infrared radiation (IR) thermometer. An exemplary such device is disclosed in US-A-4,797,840.

Importantly, as with the other types of thermometers, it is necessary that there be a sanitary contact between the IR thermometer and the body during operational use. It is well known that such a sanitary contact can be accomplished in several ways. For example, the thermometer may either be sterilizable prior to a subsequent use, or used only once and then discarded. Additionally, the thermometer may somehow be protected from contact with the body. For IR thermometers which cannot be easily sterilized or are too expensive to be simply thrown away the only practical way to insure subsequent sanitary uses of the thermometer may be to provide a disposable cover or barrier for that part of the thermometer which comes into contact with the body.

Typically, in order to determine the temperature of a human body, IR thermometers use a hollow probe, or speculum, to establish a radiation pathway between a selected body surface and the thermal radiation detecting element of the thermometer. It is this probe which is normally inserted into the ear canal of the patient or otherwise brought into contact with a body surface. Thus, it is the probe of an IR thermometer which needs to be protected from contamination.

Several probe covers have been suggested to accomplish the purpose of providing a sanitary barrier between a patient and the probe of a thermometer. In addition to the sanitary barrier function, these devices typically must also fulfill up to three additional functions. First, the probe cover must typically be transparent to radiation having a wavelength in the far infrared range, i.e. at least part of the probe cover must function as an infrared window. Also, the probe cover must securely attach to the thermometer housing, and it should provide for the easy and comfortable insertion of the probe into a body cavity (e.g. the ear canal).

Previous probe covers have typically used separate components to meet all four requirements, or have not fully met one or more of these requirements. As an example of a probe cover which joins separate components to perform the various probe cover functions discussed above, US-A 4,662,360 discloses a sanitary protective cover, as defined in the precharacterizing portion of independent claim 1, for the ear canal probe of a tympanic thermometer. The US-A-4,662,360 device, as disclosed, is a multi-part cover which incorporates an essentially rigid side wall that serves as a speculum. One part (the film) of the US-A-4,662,360 device is used as the infrared window and the other part (the tube) of the US-A-4,662,360 device is used to fulfill the other three requirements discussed above.

Similarly, US-A-4,911,559 discloses a three-piece probe cover that has a stretchable film which serves as a sanitary barrier, an infrared window and a probe (speculum) envelope to facilitate insertion of the probe into the ear. The device has a separate rigid ring for securely fitting the probe cover onto the probe.

Still another probe cover is disclosed in US-A-4,790,324. It has a tubular body much thicker than a window transparent to infrared radiation and disposed across the outer end of the body. The inner tube end surrounds but is spaced from the probe.

A further multi-part probe cover is disclosed in the US-A-4,159,766. The cover has a hollow tube and a tip mounted at the forward end of the tube. The tip has a thermal window in its circumferential wall.

Unfortunately, such multi-part covers require the accomplishment of numerous tasks during the manufacturing procedure. Additionally, when the various parts of a multi-part probe cover are assembled, seams are created along the boundaries of the interconnected parts which, if they protrude excessively from the probe cover, can be uncomfortable for the patient. Perhaps more importantly, the integrity, and therefore the sanitary efficacy, of the probe cover is likely to be more easily compromised along a seam than in the other surface areas of the cover.

Thus, it is desirable that the probe cover be of unitary construction. One such probe cover is disclosed in US-A-3,949,740, which discloses a rigid probe cover which covers all but the distal end of the probe. Thus, this known probe cover establishes an infrared window for its associated probe by not covering the distal end of the probe at all. Unfortunately, because the US-A-3,949,740 device does not cover the distal end of the probe, the distal end may potentially become contaminated during use. Accordingly, there is still a need to provide a unitary probe cover which covers substantially all of the probe of a thermometer to establish an effective sanitary barrier.

The present invention recognizes that an effective unitary probe cover for an IR thermometer can be produced without requiring the assembly of various probe cover parts during the manufacturing process. Further, the present invention recognizes that a sanitary probe cover need not function as a speculum in order to accomplish its protective function.

Accordingly, it is an object of the present invention to provide a probe cover which is of unitary construction. Still another object of the present invention to provide a probe cover which establishes a continuous integrally formed unitary barrier between the probe and the surface of the body which would otherwise come into contact with the probe. Finally it is an object of the present invention to provide a probe cover that is relatively easy to use and cost-effective to manufacture.

In accordance with the invention to achieve this there is provided a cover for the hollow probe on a radiation detecting thermometer with said probe having a proximal portion at said thermometer and a distal opening at its opposite end, which cover comprises a hollow sheath having a side wall composed of a proximal portion followed by a distal portion, an open end of said proximal portion integral with which is a base engaged therewith for mounting said sheath on said probe and a closed end at said distal portion and in use disposed across said distal opening as a window, said closed end and said distal portion being thinner than said proximal portion and said base end, said closed end being sufficiently thin to be effectively transparent for transmission of infrared radiation through said distal opening, characterized in that said distal portion is of the same thickness as the said closed end and is integrally formed therewith so that said cover is a unitary one-piece member.

The preferred embodiment of a probe cover for an IR thermometer in accordance with the present invention includes a substantially hollow, frustum-shaped sheath having an open end and a closed end. The sheath is made from a single unitary piece of infrared transparent film which is sufficiently thin at the closed end of the sheath to establish an infrared window. The open end of the sheath is defined by its periphery, i.e. the edge of the piece of film. As indicated, the sheath has a frustum-shaped wall that connects the open end of the sheath with the closed end and effectively defines the shape of the sheath. The thickness of the wall may be either uniform or tapered from a relatively thick configuration at the open end of the sheath to a relatively thin configuration near the closed end of the sheath.

The base is ring-shaped and is integrally formed around and projects outwardly from the periphery of the open or proximal end of the sheath. The base is dimensioned to surroundingly engage and thereby establish an interference or interlocking fit with the outside of the hollow probe of an IR thermometer when the cover is placed over the probe. Importantly, when the base of the probe cover is engaged with the thermometer, the sheath effectively surrounds the probe and the infrared window of the sheath is positioned over the distal or free end of the probe. Thus, the window is disposed over, or across, an open pathway which extends through the probe and along which infrared radiation from inside the ear canal can be transmitted to the thermal detecting elements of the thermometer. In the construction of the unitary probe cover, both the base and the proximal portion of the wall of the sheath are relatively thick and rigid, while the IR window and the adjacent distal portion of the wall of the sheath are relatively thin and essentially flaccid. Preferably, the entire probe cover is made of a polymer, such as polyethylene or polypropylene.

Other advantageous features of the present invention are set forth in the dependent claims. The organization and manner of operation of two specific embodiments of the invention, together with further features and advantages thereof, may best be understood by reference to the following description taken in connection with the accompanying drawings, in the several figures of which like reference numerals identify like elements, and in which:
Figure 1 is a perspective view of an IR thermometer with the probe cover of the present invention engaged to cover the probe of the thermometer;
Figure 2A is a cross-sectional view of the probe cover engaged with a portion of the thermometer probe as seen along the line 2-2 in Figure 1;
Figure 2B is a cross-sectional view of an alternate embodiment of the probe cover of the present invention showing the formation of a snap ring, as would be seen along the line 2-2 in Figure 1;
Figure 3 is an elevational view of a plurality of nested probe covers;
Figure 4A is an isometric view of the probe cover of the present invention shown in Figure 2A; and
Figure 4B is an isometric view of the alternate embodiment of the probe cover of the present invention shown in Figure 2B.

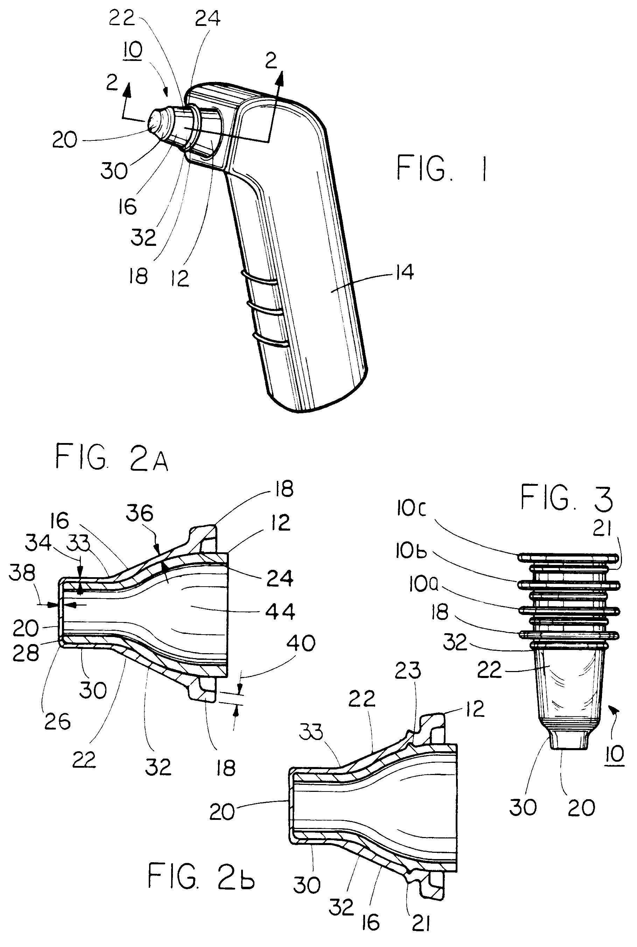

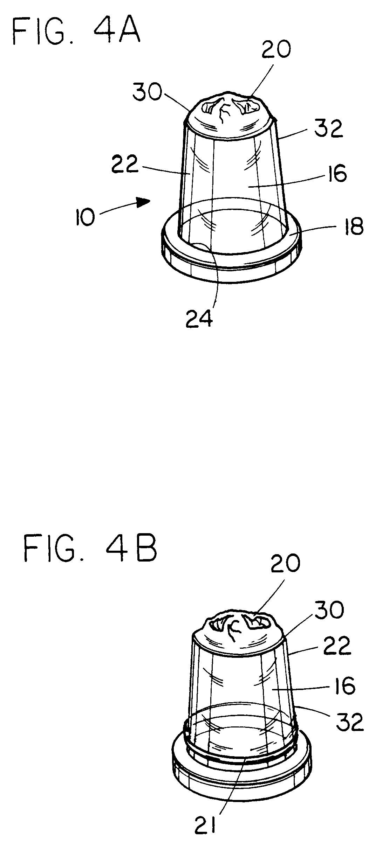

Referring initially to Figure 1, the probe cover according to the present invention is generally designated 10 and is shown positioned over the hollow probe 12 of an IR thermometer 14. In this instance, probe 12 is in the form of a speculum shaped and sized for insertion within the ear canal. Cover 10 includes a hollow sheath 16 that is unitarily formed with a stiff radially-thicker circular base ring 18 at one end adjacent or approximate to thermometer 14. More specifically, sheath 16 and base ring 18 are formed from a single piece of material which does not readily absorb light having wavelengths in the far infrared range (approximately four to twenty microns). Polyethelyne or polypropylene (for instance, polyolefin or a suitable co-polymer) are preferred. When sheath 16 is positioned on probe 12 the distal or closed end of sheath 16 forms a flat, generally circular, wrinkle-free infrared transparent window 20. Also as shown in Figures 2A and 4A, wall 22 of sheath 16 is shaped as a right circular frustrum that extends between window 20 and the periphery 24 of base ring 18.

Several embodiments of the probe cover are contemplated by the present invention and two of them are described in detail. First as seen in Figures 2A and 4A the preferred embodiment of the present invention has a substantially rigid base ring 18 which is dimensioned for surrounding engagement with probe 12. More particularly, base ring 18 establishes a secure fit on probe 12. With this fit, ring 18 holds cover 10 on probe 12 so that window 20 of sheath 16 is positioned and stretched directly across the distal opening 26 of the probe tip 28.

The radial thickness of base ring 18 may vary from 0.38 to 1.27 mm [fifteen thousandths (0.015) to fifty thousandths (0.050) of an inch] depending on the particular application of probe 12, while window 20 has a thickness of between 0.025 and 0.012 mm (0.001 and 0.0005 inch) when using one of the materials mentioned above. The thickness is selected to be as thin as possible to minimize attenuation of the radiation while yet remaining sufficiently strong to resist being torn or distorted during use. Also, as shown, wall 22 of sheath 16 extends between window 20 and base ring 18 to cover a substantial portion of the probe 12. Wall 22 is virtually a continuation of ring 18 and its thickness in this case is established to be between 0.072 and 0.76 mm [five ten thousandths (.0005) and thirty thousandths (.030) of an inch]. To provide additional stiffness to wall 22, several elongated ribs (not shown) may be formed along wall 22.

Alternatively, as shown in Figures 2B and 4B, wall 22 of sheath 16 is formed to include an offset 21 that defines an internal circular recess and an external circular rib to enable base ring 18 to function as a snap ring. Offset 21 circumscribes wall 22 and is dimensioned to surroundingly engage probe 12. More specifically, the recess of offset 21 forms an interlocking fit with a circular nipple 23 formed on probe 12. Nipple 23 may be in the form of a plurality of protrusions which are raised on probe 12 or a continuous raised ring which circumscribes probe 12.

In either embodiment, wall 22 includes, in axial order from window 20 to periphery 24, a distal portion 30 and a proximal portion 32. The shape of proximal portion 32 of wall 22 is adapted for comfortable insertion into a body cavity, such as an auditory canal. As disclosed above, window 20 has a thickness 38 of between approximately 0.025 and 0.012 mm [one thousandth (0.001) and five ten thousandths (.0005) of an inch]. Consequently, window 20 is sufficiently thin to be effectively infrared transparent. Additionally, distal portion 30 of wall 22 has a thickness 34 which is also between approximately 0.025 and 0.012 mm [one thousandth (0.001) and five ten thousandths (.0005) of an inch]. The proximal portion 32 of wall 22, on the other hand, has a thickness 36 which is approximately 0.5 mm [twenty thousandths (0.020) of an inch].

In the preferred form, wall 22 is gradually tapered from approximately 0.5 mm [twenty thousandths (.020) of an inch] in proximal portion 32 to a thickness of approximately 0.012 mm [five ten thousandths (.0005) of an inch] in distal portion 30. However, proximal portion 32 of wall 22 can have a thickness as great as 0.76 mm [thirty thousandths (.030) of an inch]. Accordingly, the relatively thick base 18 and the relatively thick proximal portion 32 of wall 22 are both substantially stiffer than window 20 and distal portion 30 of wall 22, both of which are relatively thinner and substantially flaccid. Preferably, the length of the tapered portion 33 of wall 22 is at least ten times the difference between the thickness 36 of proximal portion 32 and the thickness 34 of distal portion 30. This gradual tapering has several advantages, such as better tear-resistance, better patient comfort and ease of manufacturing.

In another embodiment, wall 22 can be effectively stiffened along most of its length by being made to have a substantially uniform thickness (e.g. 0.020 in). With a stiff wall 22, cover 10 may also be useful as a speculum. In either case, the thickness 40 of base ring 18 and thickness 36 of proximal portion 32 are sufficient to provide a structural stiffness for securely attaching cover 10 to probe 12. The thickness 38 of window 20, however, remains approximately 0.012 - 0.025 mm [five to ten ten thousandths (.0005 - .0010) of an inch] to establish an IR transparent window. In a further alternative, the entire length of wall 22 is made to be flaccid, i.e. wall 22 would have a thickness of 0.012 mm [five ten thousandths (.0005) of an inch] or greater from base ring 18 to window 20.

Because of the varying thickness of the material of probe cover 10, the general frustum shape of probe cover 10 is established and maintained by proximal portion 32, while the substantially circular shape of periphery 24 is established and maintained by base ring 18. On the other hand, because window 20 and distal portion 30 are effectively flaccid for the preferred embodiment of cover 10, they can readily conform to the contour of probe 12 when cover 10 is fitted over the probe. Indeed, when cover 10 is properly positioned over probe 12, window 20 and distal portion 30 of wall 22 are drawn tightly against the outer surface of probe 12 to create a sanitary barrier over the probe 12.

Importantly, window 20 and distal portion 30 of the cover 10 are substantially wrinkle-free when the cover 10 is properly positioned over probe 12.

The inclusion of thin distal portion 30 ensures that a thin, transparent portion of sheath 16 is positioned to serve as window 20 even should cover 10 somehow be mounted askew. Distal portion 30 also enables cover 10 to fit various probes or specula of differing distal portion diameter or length. At the same time, manufacturing tolerances may be relaxed. As intended for the present invention and as indicated above, the entire cover 10, including base ring 18, window 20, and wall 22, is made from a single continuous piece of material. As indicated above, this material is a material such as polyethylene or polypropylene (for instance, polyolefin or a suitable co-polymer are preferred). Regardless of the particular material used for the manufacture of cover 10, however, it is important that the window 20 be sufficiently thin to be effectively transparent to the transmission of infrared energy. Further, in order to obtain consistent results and minimize any attenuation of the infrared radiation which will pass through window 20, window 20 should preferrably be as flat and wrinkle-free as possible when fitted onto probe 12, as disclosed above.

Referring for the moment to Figure 3, a method for the storage of several probe covers 10, respectively designated 10a, 10b and 10c, is shown. Specifically, because of the dimensions of wall 22 of sheath 16, the probe covers 10, 10a, 10b, 10c, can be nested, as shown for the purpose of space saving.

First, probe cover 10 is engaged with probe 12. More particularly, for the embodiment shown in Figures 2A and 4A, base ring 18 or the substantially stiff proximal portion 32 of wall 22, or both, engage probe 12 to form an interference or friction fit with probe 12. On the other hand, for the embodiment shown in Figures 2B and 4B, the recess of offset 21 engages nipple 23 to form an interlocking fit. With cover 10 positioned on probe 12 of thermometer 14 as shown in Figure 1, it will be appreciated that sheath 16 serves as a sanitary barrier. Specifically, sheath 16 acts as a barrier which protects a patient from direct contact with probe 12 and probe tip 28 when these parts of thermometer 14 otherwise would come into physical contact with the patient (not shown). Although sheath 16 is an effective sanitary barrier, infrared radiation from a body surface, such as the inside of the patient's ear canal, can still pass through window 20 of sheath 16 and on through channel 44 of probe 12 for detection and display by thermometer 14.

## Claims

1. Cover for the hollow probe on a radiation detecting thermometer (14) with said probe (12) having a proximal portion at said thermometer (14) and a distal opening at its opposite end, which cover (10) comprises a hollow sheath (16) having a side wall (22) composed of a proximal portion (32) followed by a distal portion (30), an open end of said proximal portion (32) integral with which is a base (18) engaged therewith for mounting said sheath (16) on said probe (12) and a closed end at said distal portion (30) and in use disposed across said distal opening as a window (20), said closed end and said distal portion (30) being thinner than said proximal portion (32) and said base end, said closed end being sufficiently thin to be effectively transparent for transmission of infrared radiation through said distal opening, characterized in that said distal portion (30) is of the same thickness as the said closed end and is integrally formed therewith so that said cover (10) is a unitary one-piece member.

2. Cover according to claim 1, characterized in that said proximal portion (32) is frustum-shaped and is made of a polymer material.

3. Cover according to claim 1 or 2, characterized in that said probe (12) has an infrared transparent end and said window (20) stretchably covers said end.

4. Cover according to claim 1, characterized in that said side wall (22) connects said window (20) with said base (18), and said proximal portion (32) tapering with increasing thickness from said distal portion (30) in the direction toward said base (18).

5. Cover according to claim 1, characterized in that said side wall (22) connects said window (20) with said base (18), said distal portion (30) and said window (22) having a first thickness and said proximal portion (32) having a thickness greater than said first thickness.

6. Cover according to any one of claims 1 to 5, characterized in that said base (18) and said proximal portion (32) of said wall (22) are stiff as compared with said distal portion (30).

7. Cover according to any one of claims 1 to 6, characterized in that said window (20) and said distal portion (30) are flaccid as compared with said proximal portion (32).

8. Cover according to any one of claims 1 to 7, characterized in that said base (18) has a configuration that establishes an interference fit with said probe (12).

## Patentansprüche

1. Kappe für den hohlen Meßfühler eines Strahlungserfassungsthermometers (14), wobei der Meßfühler (12) einen proximalen Teil an dem Thermometer (14) und eine distale Öffnung an seinem entgegengesetzten Ende hat, wobei die Kappe (10) eine hohle Hülle (16) aufweist, die eine Seitenwand (22) hat, welche aus einem proximalen Teil (32) gefolgt von einem distalen Teil (30) besteht, wobei an einem offenen Ende des proximalen Teils (32) ein Fuß (18) angeformt ist zum Befestigen der Hülle (16) auf dem Meßfühler (12), und ein geschlossenes Ende an dem distalen Teil (30), welches im Gebrauch als ein Fenster (20) über der distalen Öffnung angeordnet ist, wobei das geschlossene Ende und der distale Teil (30) dünner sind als der proximale Teil (32) und das Fußende und wobei das geschlossene Ende ausreichend dünn ist, um für das Durchlassen von Infrarotstrahlung durch die distale Öffnung effektiv transparent zu sein, dadurch gekennzeichnet, daß der distale Teil (30) dieselbe Dicke hat wie das geschlossene Ende und integral mit demselben ausgebildet ist, so daß die Kappe (10) ein unitäres einstückiges Teil ist.

2. Kappe nach Anspruch 1, dadurch gekennzeichnet, daß der proximale Teil (32) kegelstumpfförmig ist und aus einem Polymermaterial besteht.

3. Kappe nach Anspruch 1 oder 2, dadurch gekennzeichnet, daß der Meßfühler (12) ein infrarottransparentes Ende hat und daß das Fenster (20) das Ende dehnbar bedeckt.

4. Kappe nach Anspruch 1, dadurch gekennzeichnet, daß die Seitenwand (22) das Fenster (20) mit dem Fuß (18) verbindet und daß der proximale Teil (32) mit zunehmender Dicke von dem distalen Teil (30) in Richtung zu dem Fuß (18) konisch zuläuft.

5. Kappe nach Anspruch 1, dadurch gekennzeichnet, daß die Seitenwand (22) das Fenster (20) mit dem Fuß (18) verbindet, wobei der distale Teil (30) und das Fenster (22) eine erste Dicke haben und wobei der proximale Teil (32) eine Dicke hat, die größer als die erste Dicke ist.

6. Kappe nach einem der Ansprüche 1 bis 5, dadurch gekennzeichnet, daß der Fuß (18) und der proximale Teil (32) der Wand (22) im Vergleich zu dem distalen Teil (30) steif sind.

7. Kappe nach einem der Ansprüche 1 bis 6, dadurch gekennzeichnet, daß das Fenster (20) und der distale Teil (30) im Vergleich zu dem proximalen Teil (32) lappig sind.

8. Kappe nach einem der Ansprüche 1 bis 7, dadurch gekennzeichnet, daß der Fuß (18) eine Konfiguration hat, die mit dem Meßfühler (12) einen Festsitz ergibt.

## Revendications

1. Capot pour la sonde creuse d'un thermomètre de mesure de radiation (14), cette sonde (12) comportant une partie proximale du côté du thermomètre (14) et une ouverture distale à son extrémité opposée, le capot (10) comprenant une gaine creuse (16) ayant une paroi latérale (22) constituée d'une partie proximale (32) suivie d'une partie distale (30), une extrémité ouverte de ladite partie proximale (32) comportant une base (18) destinée au montage de la gaine (16) sur la sonde (12), et une extrémité fermée à l'endroit de la partie distale (30) et qui, en cours d'utilisation, est disposée de l'ouverture distale, comme une fenêtre (20), l'extrémité fermée et la partie distale (30) étant plus minces que la partie proximale et base, l'extrémité fermée étant suffisamment mince pour pouvoir être transparente à une radiation infrarouge traversant l'ouverture distale, caractérisé en ce que la partie distale (30) a la même épaisseur que l'extrémité fermée et elle est formée d'une manière intégrale avec celle-ci de telle façon que le capot (10) constitue un élément unitaire d'une seule pièce.

2. Capot suivant la revendication 1, caractérisé en ce que la partie proximale (32) est en forme de tronc de cône et est constituée d'un matériau polymère.

3. Capot suivant la revendication 1 ou 2 caractérisé en ce que la sonde (12) comporte une extrémité transparente à une radiation infrarouge et la fenêtre (20) recouvre cette extrémité d'une manière étirable.

4. Capot suivant la revendication 1 caractérisé en ce que la paroi latérale (22) relie la fenêtre (20) à la base (18) et la partie proximale tronconique a une épaisseur allant en augmentant de la partie distale (30) vers ladite base (18).

5. Capot suivant la revendication 1 caractérisé en ce que la paroi latérale (22) relie la fenêtre (20) à la base (18), la partie distale (30) et la fenêtre (20) ayant une première épaisseur, et la partie proximale (32) ayant une épaisseur supérieure à cette première épaisseur.

6. Capot suivant l'une quelconque des revendications 1 à 5 caractérisé en ce que la base (18) et la partie proximale (32) de la paroi (22) sont rigides en comparativement à la partie distale (30).

7. Capot suivant l'une quelconque des revendications 1 à 6 caractérisé en ce que la fenêtre (20) et la partie distale (30) sont souples comparativement à de la partie proximale (32).

8. Capot suivant l'une quelconque des revendications 1 à 7 caractérisé en ce que la base (18) a une configuration qui établit un ajustement étroit avec la sonde (12).
